# EUROPEAN PATENT SPECIFICATION

(11) **EP 4 473 045 B1**
(45) Date of publication and mention of the grant of the patent: **22.04.2026**
(21) Application number: 22706156.1
(22) Date of filing: 03.02.2022
(51) Int. Cl.: C08J 9/08, A61B 10/00, A61F 5/453, A61F 5/455

(54) **EXTERNAL CATHETER WITH IMPROVED HAPTIC FEEDBACK AND RELATED SYSTEMS AND METHODS**
EXTERNER KATHETER MIT VERBESSERTER HAPTISCHER RÜCKMELDUNG SOWIE ZUGEHÖRIGE SYSTEME UND VERFAHREN
CATHÉTER EXTERNE À RÉTROACTION HAPTIQUE AMÉLIORÉE ET SYSTÈMES ET PROCÉDÉS ASSOCIÉS

(43) Date of publication of application: 11.12.2024
(73) Proprietor: Purewick Corporation, El Cajon, California 92020 (US)
(72) Inventor: ROTHBERG, Matthew Jordan, Atlanta, Georgia 30306 (US); BAILEY, Michael James, Creedmoor, North Carolina 27522 (US); ESPOSITO, Anthony, Oxford, Georgia 30054 (US); REED, Jeffrey, Minden, New Mexico 68959 (US); SCHNEIDER, Seth, Social Circle, Georgia 30025 (US)
(74) Representative: Hoffmann Eitle
(86) International application number: PCT/US2022/015045
(87) International publication number: WO 2023/149882

(56) References cited:
- EP-A1- 0 966 936
- EP-A1- 0 987 293
- GB-A- 2 260 907
- US-A- 5 340 840
- US-A1- 2016 374 848
- US-A1- 2018 228 642

## Description

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bedpans and urinary catheters have several problems associated therewith. For example, bedpans may be prone to discomfort, spills, and other hygiene issues. Urinary catheters be may be uncomfortable, painful, and may cause urinary tract infections. Conventional fluid collection devices also may be limited to use when a patient is confined to a bed in a supine position.

Thus, users and manufacturers of fluid collection devices continue to seek new and improved devices, systems, and methods to collect fluid.

US 2018/228642 A1 discloses in combination the technical features of the precharacterizing portion of claim 1 below.

### SUMMARY

The present invention is defined in claim 1, below. The dependent claims are directed to optional features and preferred embodiments of the invention. Embodiments disclosed herein are related to fluid collection systems and methods of manufacturing fluid collection systems. In an embodiment, a fluid collection device includes a fluid impermeable barrier at least partially defining a chamber, the fluid impermeable barrier also defining an opening extending therethrough, the opening configured to be positioned adjacent to a urethra of a user. The fluid impermeable barrier includes at least one foaming agent incorporated therein, the at least one foaming agent being composed to increase a flexibility of the fluid impermeable barrier and maintain a selected geometric configuration. The fluid collection device further includes a conduit having an inlet and an outlet and may also include a permeable material disposed in the chamber. The inlet is positioned in the chamber and the outlet extends through an aperture in the fluid impermeable barrier.

In an embodiment, a fluid collection system may include a fluid storage container configured to hold a fluid and a fluid collection device fluidly coupled to the fluid storage container, which fluid collection device includes a fluid impermeable barrier at least partially defining a chamber. The fluid impermeable barrier also defines an opening extending there through, the opening configured to be positioned adjacent to a urethra of a user. The fluid impermeable barrier includes at least one foaming agent incorporated therein, the at least one foaming agent being composed to increase a flexibility of the fluid impermeable barrier and maintain a selected geometric configuration. The fluid collection device also includes a permeable material disposed in the chamber. The fluid collection system also includes a conduit having an inlet and an outlet. The outlet is fluidly coupled to the fluid storage container and the inlet is positioned in the chamber. The fluid collection system may also include a vacuum source configured to draw fluid from the fluid collection device.

A method to manufacture such a fluid collection device is disclosed. The method includes providing a raw material mixture and shaping the raw material mixture into a fluid impermeable barrier. The method also may include incorporating the fluid impermeable barrier into the fluid collection device.

Features from any of the disclosed embodiments may be used in combination with one another, without limitation. In addition, other features and advantages of the present invention will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
**FIG. 1A** is a block diagram of a portable fluid collection system.
**FIG. 1B** is an isometric view of a fluid collection system.
**FIG. 2A** is an isometric view of a female fluid collection device.
**FIG. 2B** is across sectional view of the fluid collection device of **FIG. 2A****.**
**FIG. 3A** is a cross sectional view of a male urine collection assembly.
**FIG. 3B** is an isometric view of a male urine collection assembly.
**FIG. 3C** is a cross sectional view of the male urine collection assembly of **FIG. 3B****.**
**FIG. 4** is a flow diagram of a method to manufacture a fluid collection device, according to the invention.

### DETAILED DESCRIPTION

Embodiments disclosed herein are related to fluid collection systems and related methods. The devices and systems disclosed herein are configured to collect fluids from an individual. The fluids collected by the fluid collection devices may include at least one of urine, vaginal discharge, penile discharge, reproductive fluids, blood, sweat, or other bodily fluids. Many users of fluid collection devices are over 65 years old with limited mobility, often relying on wheelchairs as a primary mode of transportation. Many users also have medical conditions that may require them to be in a sitting or supine position for a long duration of time. Because of this immobility, the fluid collection device may be situated in the groin area and in contours of the anatomy of a wearer in a region proximate the urethra of the user and in place for a long period of time. Users and caregivers, then, are benefited from a fluid collection device that may be improve the haptic qualities of the device and/or be capable of a more comfortable experience using the device without affecting the capabilities of the system, allowing users and/or caregivers to use the urine collection system to collect fluid with greater comfort than other comparable devices and/or systems.

The addition of at least one foaming agent to components of the fluid collection system may broaden the range of the feel and/or deflection of the components. In some embodiments, the material properties and function of the components may remain unaffected by the addition of the foaming agent, while comfort level for the user may be vastly improved. Prior methods for varying haptic user experience has been attempted by varying material type, thicknesses, and/or geometry. Embodiments of the fluid collection systems described herein allow for a reasonable component thickness while allowing for an effectively softer feel for the benefit of the user and/or caregiver.

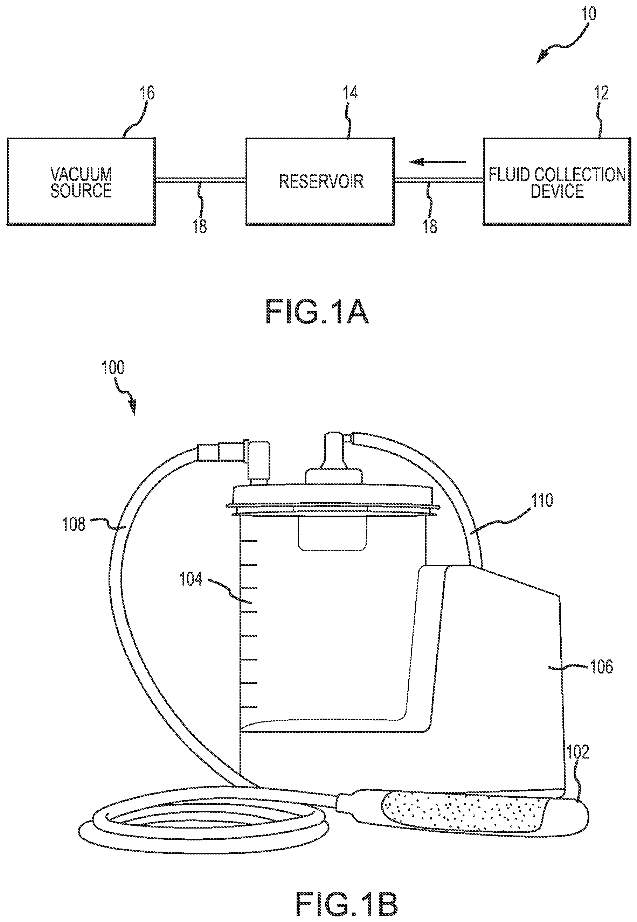

**FIG. 1A** is a block diagram of a fluid collection system 10. The fluid collection system 10 may be included in embodiments of fluid collection systems described herein. The system 10 includes a fluid (*e.g.,* urine) collection device 12 (*e.g.,* any of the fluid collection assemblies disclosed herein), a fluid collection container 14 (or reservoir), and a vacuum source 16 (or pump). The fluid collection device 12, the fluid collection container 14, and the vacuum source 16 may be fluidly coupled to each other via one or more conduits 18. For example, fluid collection device 12 may be operably coupled to one or more of the fluid collection container 14 or the vacuum source 16 via the conduit 18. In some embodiments, the vacuum source 16 may be coupled directly to the fluid collection container 14. Fluid (*e.g.,* urine or other bodily fluids) collected in the fluid collection assembly 12 may be removed from the fluid collection assembly 12 via the conduit 18 coupled to the fluid collection device 12. Suction force may be introduced into a chamber of the fluid collection device 12 via the inlet of the conduit 18 responsive to suction (*e.g*., vacuum) force applied at the outlet of the conduit 18.

The suction force may be applied to the outlet of the conduit 18 by the vacuum source 16 either directly or indirectly. The suction force may be applied indirectly via the fluid collection container 14. For example, the outlet of the conduit 18 may be disposed within or fluidly coupled to an interior region of the fluid collection container 14 and an additional conduit 18 may extend from the fluid collection container 14 to the vacuum source 16. Accordingly, the vacuum source 16 may apply suction to the fluid collection assembly 12 via the fluid collection container 14. The suction force may be applied directly via the vacuum source 16. For example, the outlet of the conduit 18 may be disposed within the vacuum source 16 (e.g. pump). An additional tube 18 may extend from the vacuum source 16 to a point outside of the fluid collection device 12, such as to the fluid collection container 14. In such examples, the vacuum source 16 may be disposed between the fluid collection device 12 and the fluid collection container 14.

The fluid collection container 14 may be sized and shaped to retain a fluid therein. The fluid collection container 14 may include a bag (*e.g*., drainage bag), a bottle or cup (*e.g.,* collection jar), or any other enclosed container for storing bodily fluid(s) such as urine. In some examples, the conduit 18 may extend from the fluid collection device 12 and attach to the fluid collection container 14 at a first point therein. An additional conduit 18 may attach to the fluid collection container 14 at a second point thereon and may extend and attach to the vacuum source 16. Accordingly, a vacuum (*e.g*., suction) may be drawn through fluid collection device 12 via the fluid collection container 14. Fluid, such as urine, may be drained from the fluid collection device 12 via the conduit 18 using the vacuum source 16.

The vacuum source 16 or pump may include one or more of a manual vacuum pump, and electric vacuum pump, a diaphragm pump, a centrifugal pump, a displacement pump, a magnetically driven pump, a peristaltic pump, or any pump configured to produce a vacuum. The vacuum source 16 may provide a vacuum or suction to remove fluid from the fluid collection device 12. In some examples, the vacuum source 16 may be powered by one or more of a power cord (*e.g.,* connected to a power socket), an alternator, one or more batteries, or even manual power (*e.g.,* a hand operated vacuum pump). The vacuum sources disclosed herein may include one or more of a switch, a button, a plug, a remote, or any other device suitable to activate the vacuum source 16.

Many embodiments of fluid collection systems described herein are configured to be worn by a user, positioned on a surface such as a table, and/or securable or mountable to a wheelchair. **FIG. 1B** is an isometric view of a fluid collection system 100. The fluid collection system 100 may include a fluid collection device 102 configured to receive fluid (e.g. urine) from a user. The fluid collection device 102 may be removably attached to a fluid collection container 104 of the fluid collection system 100. The fluid collection container 104 may be integrated into a housing 106 that may also include a vacuum device (e.g. a pump) therein. The fluid collection system 100 may further include a collection conduit 108 and a suction conduit 110. The collection conduit 108 may be disposed between the fluid collection device 102 and the fluid collection container 104. The collection conduit 108 may fluidly connect the internal volume of the fluid collection container 104 with the fluid collection device 102. The suction conduit 110 may couple the pump suction and/or discharge to the fluid collection container 104. The pump disposed within the housing 106 may be in fluid communication with the internal volume of the fluid collection container 104 via the suction conduit 110. The housing 106 may also be configured to store or house internal electrical and mechanical components. For example, the housing may include a pump (not shown in **FIG. 1B**) configured to modulate pressure within the container 104.

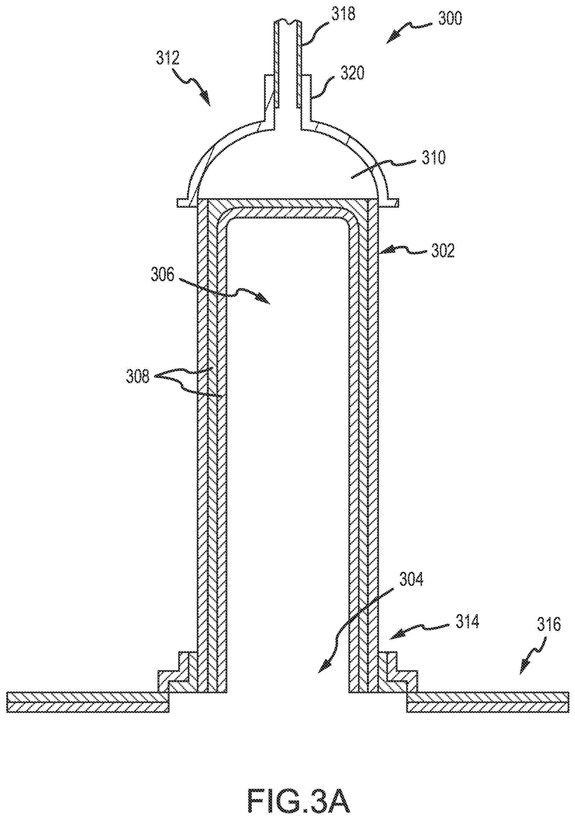

The fluid collection device 102 may be positioned at least proximate to a urethral opening. The fluid collection device shown in **FIGS. 1B-2B** are examples of female fluid collection devices configured to collect fluid(s) from females (*e.g*., collect urine from a female urethra). Further examples of female fluid collection assemblies are disclosed in U.S. Patent No. 10,390,989 issued on August 27, 2019. However, the fluid collection assemblies, systems, and methods disclosed herein may include male fluid collection assemblies and/or devices shaped, sized, and otherwise configured to collect fluid(s) from males (*e.g*., collect urine from a male urethra). **FIGS. 3A-3C** are examples of male fluid collection devices configured to collect fluid(s) from males. Further examples of male fluid collection assemblies are disclosed in U.S. Provisional Patent Applications No. 63/067,542 filed on August 19, 2020 and U.S. Patent Application No. 16/433,773 filed on June 6, 2019.

In some embodiments, the collection conduit 108, the suction conduit 110 and/or other tubing of the fluid collection system 100 may include a flexible material such as materials tubing (*e.g*., medical tubing). Such material tubing may include a thermoplastic elastomer, polyvinyl chloride, ethylene vinyl acetate, polytetrafluoroethylene, flexible metal, ceramic and composite material tubing etc. The collection conduit 108 may include silicon or latex. In some embodiments, the collection conduit 108 may be constructed of any suitable material to be impermeable to fluids such that fluids may be drawn from the fluid collection device 102 and into the collection conduit 108. In some embodiments, the collection conduit 108 may include one or more portions that are resilient, such as by having one or more of a diameter or wall thickness that allows the collection tube 108 to be flexible.

The fluid collection container 104 may be integrated into the housing 106. In some embodiments, the container 104 may include at least an inlet coupled to the collection conduit 108 and an outlet coupled to a vacuum port that is coupled to the vacuum source. In some embodiments, the container 104 may be opaque or clear according to different embodiments and may include a rectangular front or rear profile as shown in **FIG. 1B**.

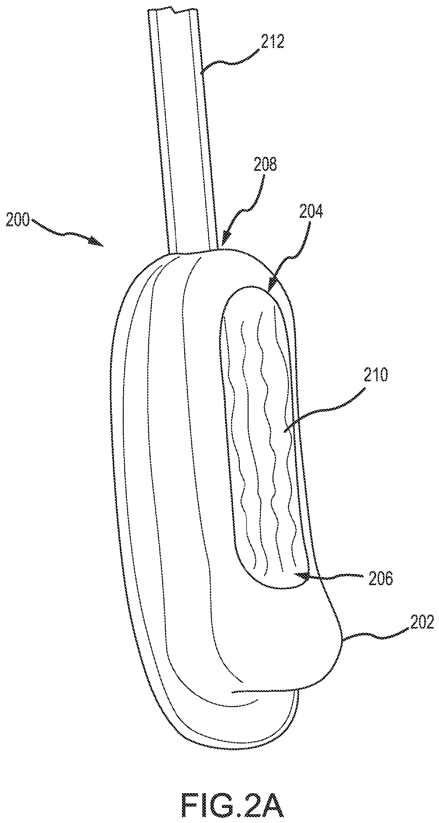

**FIG. 2A** is an isometric view of a fluid collection device 200, according to an embodiment. The fluid collection device 200 includes a fluid impermeable barrier 202, an opening 204, a chamber 206, an aperture 208 in the fluid impermeable barrier 202, and a permeable material 210 disposed in the chamber 206 within the fluid impermeable barrier 202. A conduit 212 is partially disposed within the chamber 206. The inner surfaces of the fluid impermeable barrier 202 at least partially defines the chamber 206 within the fluid collection device 200. The fluid impermeable barrier 202 temporarily stores the bodily fluids in the chamber 206.

In some embodiments, the fluid impermeable barrier 202 may be formed of a thermoplastic elastomer. Thermoplastic elastomers include a class of copolymers that include material having bother thermoplastic and elastomeric properties. In some embodiments, the thermoplastic elastomer of the fluid impermeable barrier 202 may include at least one of polyethylene, polypropylene, polyamide, and polyvinylchloride. As such, the fluid impermeable barrier 202 substantially prevents the bodily fluids from passing through the fluid impermeable barrier 202. In some examples, the fluid impermeable barrier 202 may be tubular (ignoring the opening), such as substantially cylindrical (as shown), oblong, prismatic, or flattened tubes. During use, the fluid impermeable barrier 202 may contact the wearer. The fluid impermeable barrier 202 may be sized and shaped to fit in the gluteal cleft between the legs of a female user.

To aid the comfort of the user, the fluid impermeable barrier 202 includes at least one foaming agent incorporated therein, the at least one foaming agent being composed to increase flexibility of the fluid impermeable barrier 202 and maintain a selected geometric configuration. The at least one foaming agent may also reduce the weight of the fluid collection device 200. The reduction of weight correlates to a use of less thermoplastic elastomer within the fluid impermeable barrier 202, which may provide cost savings during manufacturing. In some embodiments, the reduction of weight may include between about 15% and about 25% reduction by weight compared to a fluid impermeable barrier 202 including only the thermoplastic elastomer. In some embodiments, the reduction of weight may include about a 19% reduction. The reduction in weight may be equivalent to about 2-4 grams of the fluid impermeable barrier 202 prior to assembly into the fluid collection device 200.

In some embodiments, the fluid impermeable barrier 202 may include less than 10% by volume of the at least one foaming agent. In some embodiments, the fluid impermeable barrier 202 may include between about 0.4% and about 5% of the at least one foaming agent by volume. The foaming agent content may be selected according to desirable properties and manufacturing considerations of the fluid collection device 200. The foaming agent and the inclusion of a foaming agent may lower processing temperatures and may produce uniform cell structure while lowering the density of the fluid impermeable barrier 202. In some embodiments, the fluid impermeable barrier 202 may include a specific gravity less than 0.85. In some embodiments, the specific gravity of the fluid impermeable barrier 202 may be between 0.45 and 0.85. In other embodiments, the specific gravity of the fluid impermeable barrier 202 may be between 0.65 and 0.75.

In some embodiments, the at least one foaming agent included in the fluid impermeable barrier 202 may include a sodium bicarbonate. Sodium Bicarbonate is the monosodium salt of carbonic acid. In raw form, sodium bicarbonate appears as odorless white crystalline powder or lumps. Sodium bicarbonate decomposes at about 228°F (about 108°C). In some embodiments, the at least one foaming agent included in the fluid impermeable barrier 202 may include an azide compound. An azide includes an anion with the formula N₃⁻. The azide compound included in the fluid impermeable barrier 202 as a foaming agent may include inorganic azides such as sodium azide or organic azides such as aryl azides and/or alkyl azides. The foaming agents may be included in the fluid impermeable barrier 202 to reduce weight and improve flexibility while retaining the original properties of the thermoplastic elastomer and lowering resin usage in the method to manufacture a fluid collection device described in greater detail below.

The opening 204 of the fluid collection device 200 provides an ingress route for fluids to enter the chamber 206. The opening 204 is defined by the fluid impermeable barrier 202 such as by an inner edge of the fluid impermeable barrier 202. The opening 204 is formed in and extends through the fluid impermeable barrier 202, thereby enabling fluid(s) to enter the chamber 206 from outside of the urine collection assembly 200. The opening 204 may be an elongated hole in the fluid impermeable barrier 202. For example, the opening 204 may be defined as a cut-out in the fluid impermeable barrier 202. The opening 204 may be located and shaped to be positioned adjacent to a female urethra.

The fluid collection device 200 may be positioned adjacent to the urethra of a user and bodily fluid may enter the chamber 206 of the fluid collection device 200 via the opening 204. The fluid collection device 200 is configured to receive the fluid(s) into the chamber 206 via the opening 204. When in use, the opening 204 may have an elongated shape that extends from a first location below the urethral opening (*e.g.,* at or near the anus or the vaginal opening) to a second location above the urethral opening (*e.g.,* at or near the top of the vaginal opening). The opening 204 in the fluid impermeable barrier 202 may exhibit a length that is measured along the longitudinal axis of the fluid collection device 200. In other words, the fluid impermeable barrier 202 may define a cylindrical shape with a longitudinally extending opening therein.

The fluid collection device 200 may include at least one permeable material 210 disposed in the chamber 206. The permeable material 210 may cover at least a portion *(e.g.,* all) of the opening 204. The permeable material 210 may be exposed to the environment outside of the chamber 206 through the opening 204. The permeable material 210 may be configured to wick and/or allow flow of any fluid away from the opening 204, thereby preventing the fluid from escaping the chamber 206. The permeable properties referred to herein may be wicking, capillary action, diffusion, or other similar properties or processes, and are referred to herein as "permeable" and/or "porous." Such "wicking" and "permeable" properties may not include absorption of fluid into the permeable material 210. Put another way, substantially no absorption or solubility of the bodily fluids into the material may take place after the material is exposed to the bodily fluids and removed from the bodily fluids for a time. While no absorption or solubility is desired, the term "substantially no absorption" may allow for nominal amounts of absorption and/or solubility of the bodily fluids into the permeable material 210 (*e.g*., absorbency), such as less than about 30 wt.% of the dry weight of the permeable material 210, less than about 20 wt.%, less than about 10 wt.%, less than about 7 wt.%, less than about 5 wt.%, less than about 3 wt.%, less than about 2 wt.%, less than about 1 wt.%, or less than about 0.5 wt.% of the dry weight of the permeable material 210.

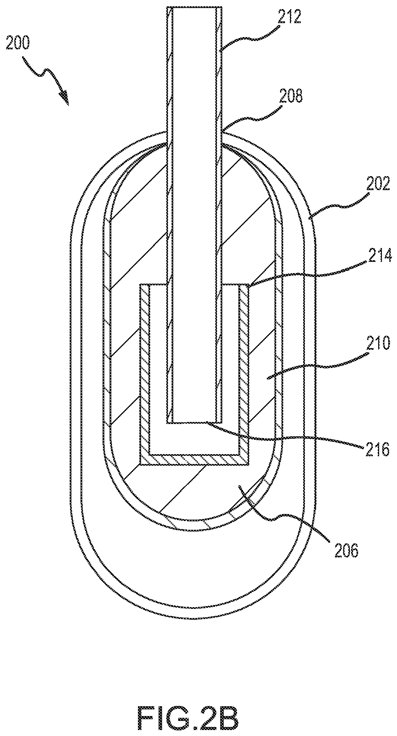

In an embodiment, the permeable material 210 may include at least one absorbent or adsorbent material. The permeable material 210 disposed within the chamber 206 may include any material that may wick and/or allow flow of the fluid. For example, the permeable material 210 may be formed from fibers from nylon (*e.g*., spun nylon fibers), polyester, polyethylene, polypropylene, wool, silk, linen, cotton (*e.g*., cotton gauze), felt, other fabrics and porous polymers, hydrophobic foam, an open cell foam polyurethane, a coated porous material (*e.g*., hydrophobic coated porous material, materials with affinity to specific substances), polymeric sintered particles from polyethylene, polypropylene, polytetrafluoroethylene(PTFE), elastomeric particles, any other suitable wicking materials, or combinations thereof. For example, the permeable material 210 may include a body of spun nylon fibers with an outer fabric gauze layers that wraps around the body of spun nylon fibers. Forming the permeable material 210 from gauze, soft fabric, and/or smooth fabric may reduce chaffing caused by the urine collection assembly 200. In some embodiments, the permeable material 210 may at least substantially and/or completely fill the portions of the chamber 206 that may not be occupied by the conduit 212.The conduit 212 is partially disposed in the chamber 206. The conduit 212 is used to remove fluid form the chamber 206. The conduit 212 may be in fluid communication with the aperture 208 of the fluid collection device 200. The conduit 212 includes an inlet (shown in **FIG. 2B**) disposed in the chamber 206 and an outlet (not shown) positioned downstream from the inlet. The outlet may be operably coupled to a fluid collection container (*e.g*. fluid collection container 104. Thus, the conduit 212 may fluidly couple the chamber 206 with the fluid collection container (as shown in **FIGS. 1A** or **1B**).

The aperture 208 of the fluid impermeable barrier 202 is sized appropriately to receive the conduit 212. The conduit 212 is disposed in the chamber 206 via the aperture 208. The aperture 208 may be sized and shaped to form an at least substantially fluid tight seal against the conduit 212, thereby substantially preventing the fluid(s) from escaping the chamber 206 via the aperture 208. As shown in **FIG. 2A**, the inlet of the conduit 212 extends through the aperture 208 and into the chamber 206. In the illustrated embodiment, the conduit 212 is at least partially disposed in the chamber 206. The fluid collected in the fluid collection device 200 may be removed from the chamber 206 via the conduit 212.

**FIG. 2B** is a cross sectional view of the fluid collection device of **FIG. 2A**, according to an embodiment. The fluid collection device 200 includes a connector 214 disposed within the chamber 206. The connector 214 couples the conduit 212 to the chamber 206 and may be disposed in the chamber 206 where the urine collects. In some embodiments, the connector 214 may be disposed within the permeable material 210. In some embodiments, the permeable material 210 fills the chamber 206 within the fluid impermeable barrier 202. The inlet 216 of the conduit 212 is coupled to the connector 214. The connector 214 may be sized and shaped to form an at least substantially fluid tight seal against the conduit 212. The connector 214 may also be coupled to the fluid impermeable barrier 202 in some embodiments. The connector 214 and the fluid impermeable barrier 202 may be coupled in a manner that forms a fluid tight seal, thereby substantially preventing the fluid(s) from escaping the chamber 206.

In some embodiments, the connector 214 may include a Shore hardness greater than the fluid impermeable barrier 202. In some embodiments, the connector 214 may include a material different than the fluid impermeable barrier 202, wherein the material includes a Shore hardness greater than the material included within the fluid impermeable barrier 202. In some embodiments, the material of the connector 214 and the fluid impermeable barrier 202 may be the same, but include a different density, hardness, or porosity than the fluid impermeable barrier 202. The connector 214 may provide a strong coupling between the conduit 212 and the other components of the fluid collection device. In some embodiments, the connector 214 may be disposed within the chamber 206 and may be disposed within the permeable material 210. Since the connector 214 is not intended to contact the skin of the user, the connector 214 may not include a foaming agent.

In this embodiment, the fluid impermeable barrier 202 is over molded onto the connector 214. Overmolding includes a multi-shot injection molding process that produces a single end product from two or more different thermoplastics. To produce the overmold, the connector 214 that includes a material having a greater Shore hardness is injection molded. Then the connector 214 may be placed in an overmold tool or overmold cavity within the same tool. The molten material that forms the fluid impermeable barrier 202 is then ejected into, onto, and/or around the connector 214. After the molten material cools, the connector 214 and the fluid impermeable barrier 202 may be chemically and/or mechanically bonded.

In some embodiments, the fluid impermeable barrier 202 and the connector 214 may be two-shot (i.e. duel-shot, multi-shot, or double-shot) molded. In some embodiments, the connector 214 may be injected into a mold to form a substrate around which the fluid impermeable barrier 202 will be molded. The substrate (e.g. connector) solidifies and cools before being transferred to a second chamber of the mold. Once the connector is in place, the material to form the fluid impermeable barrier 202 may be injected and bonded with the connector to form a bond. Then the material to form the fluid impermeable barrier 202 cools and the fluid impermeable barrier 202 and the connector 214 are then formed together.

**FIG. 3A** is a cross sectional view of a male urine collection assembly 300. In other words, the urine collection assembly 300 is designed to collect fluid(s) from male users. The urine collection assembly 300 can include a fluid impermeable barrier 302 shaped to be configured to be positioned around a penis of the user. The fluid impermeable barrier 302 is tubular or cup-shaped. The fluid impermeable barrier 302 defines an opening 304 extending through the fluid impermeable barrier 302 that is configured to have at least a portion of a penis positioned therethrough.

The fluid impermeable barrier 302 at least partially defines a chamber 306 within the urine collection assembly 300. The fluid impermeable barrier 302 temporarily stores the fluid(s) in the chamber 306. The fluid impermeable barrier 302 may be formed of any suitable fluid impermeable material(s) to substantially prevent the urine from passing through the fluid impermeable barrier 302. In some embodiments,

The fluid impermeable barrier 302 at least partially defines the chamber 306. For example, an inner surface of the fluid impermeable barrier 302 at least partially defines the perimeter of the chamber 306. Similar to the embodiments, described above in reference to **FIGS. 2A-2B**, in some embodiments, the fluid impermeable barrier 302 may include less than 10% by volume of the at least one foaming agent. The fluid impermeable barrier 302 includes between about 0.4% and about 5% of the at least one foaming agent by volume. The foaming agent content may be selected according to desirable properties and manufacturing considerations of the fluid collection device 300.

The urine collection assembly 300 may include at least one permeable material 308. The permeable material 308 may be configured to wick any fluid away from the opening 304, thereby preventing the fluid from escaping the chamber 306. The permeable material 308 may include any material that may wick the fluid. For example, the permeable material 308 may include fabric, such as a gauze (*e.g.,* a silk, linen, or cotton gauze), another soft fabric, or another smooth fabric. Forming the permeable material 308 from gauze, soft fabric, and/or smooth fabric may reduce chaffing caused by the urine collection assembly 300. The permeable material 308 may not completely fill the chamber 306 since the chamber 306 is configured to have an unoccupied space that receives at least a portion of the penis. The urine collection assembly 300 may include more than one layer of permeable material 308.

The urine collection assembly 300 may include a reservoir 310. The reservoir 310 may be a substantially unoccupied portion of the chamber 306. The fluid(s) that are in the chamber 306 may flow through the permeable material 308 to the reservoir 310. The reservoir 310 may retain the fluid(s) therein. In some embodiments, the reservoir 310 may be located at a distal end 312 of the urine collection assembly 300 and the opening may be at a proximal end 314 of the urine collection assembly. The proximal end 314 may include a base 316 that may be sized, shaped, and made of a material to be coupled to skin that surrounds the penis and have at least the urethral opening of the penis positioned therethrough. The base 316 may include the opening 304. In some embodiments, the base 316 may include a rigid structure configured to maintain the fluid impermeable membrane 302 in a particular position and/or at a particular angle relative to the individuals body (*e.g.,* the skin about the penis).

The fluid impermeable barrier 302 also includes at least a portion of a tube 318 disposed therein, such as at least partially disposed in the reservoir 310. In an example, the tube 318 may extend from a first aperture 320 at the distal region 312 to the proximal region 314 at least proximate to the opening 304. The proximal region 314 may be disposed near or on the skin around the penis (*e.g.,* on the penis or pubic area therearound). Accordingly, when a patient lays on their back, fluid (*e.g.,* urine) may aggregate near the opening 304 of the fluid impermeable barrier 302 against the skin of the subject. The fluid may be removed from the chamber 306 and/or the reservoir 310 via the tube 318. The first aperture 320 may be sized and shaped to form an at least substantially fluid tight seal against the tube 318 thereby substantially preventing the fluid(s) from escaping the chamber 306 via the first aperture 320.

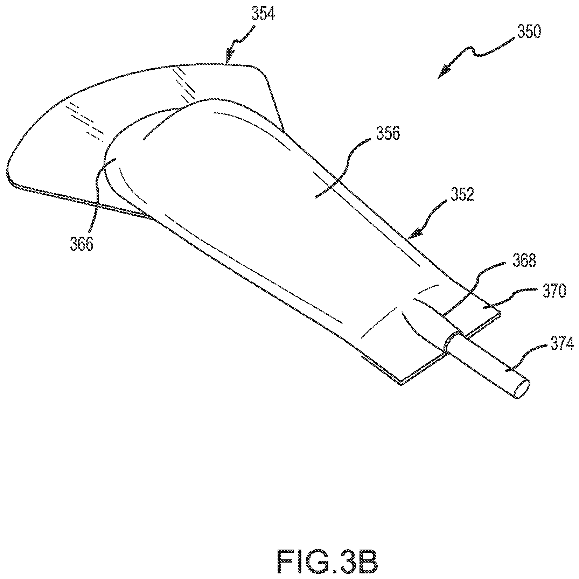

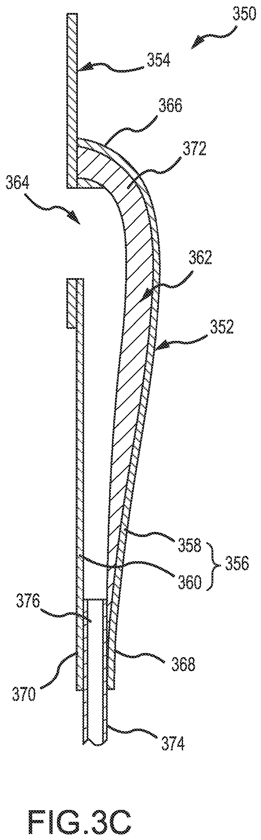

**FIG. 3B** and **FIG. 3C** are an isometric view and a cross sectional view, respectively, of a male urine collection assembly 350. The urine collection assembly 350 includes a sheath 352 and a base 354. The sheath 352 includes a fluid impermeable barrier 356 that is at least partially formed from a first panel 358 attached to a second panel 360. However, in other embodiments, the first panel 358 and the second panel 360 may be integrally formed with each other. The fluid impermeable barrier 356 also defines a chamber 362 between the first panel 358 and the second panel 360, an opening 364 at a proximal end region 366 of the sheath 352 and the base 354, and an outlet 368 at a distal end region 370 of the sheath 352. The sheath 352 also includes at least one permeable material 372 disposed in the chamber 362. The base 354 is permanently attached to the proximal end region 366 of the sheath 352.

In some embodiments, the permeable material 372 may be disposed between the first panel 358 and the second panel 360. The opening 364 is formed in and extends through the fluid impermeable barrier 356, thereby enabling bodily fluids to enter the chamber 362 from outside of the urine collection assembly 350. The opening 364 may be configured to receive a penis of the user and the penis can extend into the chamber 362.

The fluid impermeable barrier 356 defines the outlet 368 sized to receive a conduit tube 374. The tube 374 may be at least partially disposed in the chamber 362 or otherwise in fluid communication with the chamber 362 through the outlet 368. The outlet 368 may be sized and shaped to form an at least substantially fluid tight seal against the tube 374 thereby substantially preventing the bodily fluids from escaping the chamber 362. An inlet 376 of the tube 374 may be located at or near the distal end region 370 of the sheath 352 which is expected to be the gravimetrically low point of the chamber 362 when worn by a user. Locating the inlet 376 at or near the distal end region 370 of the sheath 352 enables the tube 374 to receive more of the bodily fluids than if the inlet 376 was located elsewhere and reduce the likelihood of pooling (*e.g*., pooling of the bodily fluids may cause microbe growth and foul odors). For instance, the bodily fluids in permeable material 372 due to capillary forces. However, the bodily fluids may exhibit a preference to flow in the direction of gravity, especially when at least a portion of the permeable material 372 is saturated with the bodily fluids. Accordingly, the inlet 376 may be located in the fluid collection assembly 350 in a position expected to be the gravimetrically low point in the urine collection assembly 350 when worn by a user.

Similar to the embodiments described above, in some embodiments, the sheath 352 and/or fluid impermeable barrier 356 may also include less than 10% by volume of the at least one foaming agent. In some embodiments, the fluid impermeable barrier 356 may include between about 0.4% and about 5% of the at least one foaming agent by volume. The foaming agent content may be selected according to desirable properties and manufacturing considerations of the fluid collection device 350.

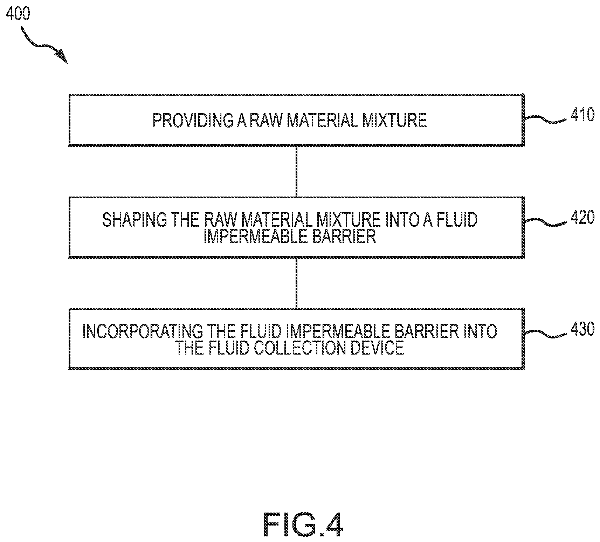

**FIG. 4** is a flow diagram of a method 400 to manufacture a fluid collection device. The method 400 includes an act 410 of providing a raw material mixture. The raw material mixture can include at least one thermoplastic elastomer and at least one foaming agent. The raw material mixture may include injection molding pellets. Act 410 may include mixing the proper ratio of thermoplastic elastomer with foaming agent to provide the proper ratio to produce a desired haptic feedback quality for a fluid impermeable barrier of the fluid collection device. The raw material mixture may include less than 10% of a foaming agent. The raw material mixture may include between about 1% and about 5% foaming agent. The foaming agent may be included in the thermoplastic elastomer injection molding pellets. In other embodiments, the foaming agent may be included as an injection molding pellet and mechanically mixed with the thermoplastic elastomer injection molding pellets.

The method 400 may further include an act 420 of shaping the raw material mixture into a fluid impermeable barrier. Shaping the raw material mixture in act 420 may include injection molding the raw material mixture. Plastic injection molding may include the process of melting plastic pellets (e.g. injection molding pellets that include the thermoplastic elastomer and/or the foaming agent) that once malleable enough, are injected at pressure into a mold cavity such as for the fluid impermeable barrier 202 described above, which fills and solidifies to produce the final product shape. The pellets may be loaded into a hopper where they can be melted, compressed, and injected into the mold runner system. The injection molding of act 420 may include heating the raw material mixture greater than 250°F to form a hot resin. The hot resin may be shot into a mold cavity and when cooled, the part is molded. Ejector pins may facilitate removal of the part from the mold.

Shaping the raw material mixture in act 420 may include extruding the raw material mixture. Similar to injection molding, the extrusion may include a raw material mixture which are melted. The extrusion molding of act 420 may include heating the raw material mixture greater than 250°F to form a hot resin. The hot resin may then be forced through a die, shaping the raw material into the final product shape (e.g. fluid impermeable barrier 202). The shape of the die determines the shape of the product. The extrusion is then cooled and forms a solid shape.

The product may include the fluid impermeable barrier of any of the embodiments described above. The fluid impermeable barrier may at least partially defining a chamber and an opening extending there through, the opening configured to be positioned adjacent to a urethra of a user. The fluid impermeable barrier includes at least one foaming agent incorporated therein, the at least one foaming agent being composed to increase a flexibility of the fluid impermeable barrier and maintain a selected geometric configuration. In some embodiments, shaping the raw material mixture in to a fluid impermeable barrier includes forming the fluid impermeable barrier into an arcuate shape conforming to a vaginal and perineal region of the wearer.

The method 400 may also include an act 430 of incorporating the fluid impermeable barrier into the fluid collection device. The fluid collection device may further include a permeable material disposed in the chamber and a conduit including an inlet and an outlet. The inlet may be positioned in the chamber and the outlet extend through an aperture in the fluid impermeable barrier. The fluid collection device further includes a connector disposed within the chamber, the inlet of the conduit being coupled to the connector, wherein the connector includes a Shore hardness greater than the fluid impermeable barrier. In accordance with the invention, the fluid impermeable barrier is over molded onto the connector.

The acts of the method of collecting fluids from a user described above are for illustrative purposes. For example, the acts of the method of collecting fluids from a user and collecting a urinalysis sample can be performed in different orders, split into multiple acts, modified, supplemented, or combined. One or more of the acts of the method of collecting fluids from a user can be omitted from the method. Any of the acts of the method of collecting a urinalysis sample from a user can include using any of the urine collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting.

## Claims

1. A fluid collection device (200), comprising:
a fluid impermeable barrier (202) at least partially defining a chamber (206), the fluid impermeable barrier also defining an opening extending therethrough, the opening configured to be positioned adjacent to a urethra of a user;
a permeable material disposed in the chamber; and
a conduit (212) including an inlet (216) and an outlet, the inlet being positioned in the chamber and the outlet extending through an aperture in the fluid impermeable barrier; and **characterized by**:
i) the fluid impermeable barrier including at least one foaming agent incorporated therein, the at least one foaming agent is composed to increase a flexibility of the fluid impermeable barrier and maintain a selected geometric configuration
ii) further comprising a connector disposed within the chamber, the inlet of the conduit being coupled to the connector, wherein the connector includes a shore hardness greater than the fluid impermeable barrier, and in that
iii) the fluid impermeable barrier is overmolded onto the connector.

2. The fluid collection device of claim 1, wherein the fluid impermeable barrier includes a thermoplastic elastomer.

3. The fluid collection device of claim 2, wherein the thermoplastic elastomer includes at least one of polyethylene, polypropylene, polyamide, and polyvinylchloride.

4. The fluid collection device of any of claims 1-3, wherein the fluid impermeable barrier includes less than 10% of the at least one foaming agent by volume.

5. The fluid collection device of any of claims 1-4, wherein the fluid impermeable barrier includes between about 0.4% and about 5% of the at least one foaming agent by volume.

6. The fluid collection device of any of claims 1-5, wherein the fluid impermeable barrier includes as specific gravity less than 0.85.

7. The fluid collection device of any of claims 1-6, wherein the at least one foaming agent includes a sodium bicarbonate.

8. The fluid collection device of any of claims 1-7, wherein the at least one foaming agent includes an azide compound.

9. The fluid collection device of any of claims 1-8, wherein the fluid impermeable barrier defines a cylindrical shape with a longitudinally extending opening therein.

10. A fluid collection system, comprising:
a fluid storage container configured to hold a fluid;
a fluid collection device as claimed in any one of the preceding claims, fluidly coupled to the fluid storage container and
a vacuum source fluidly coupled to one or more of the fluid storage container or the fluid collection device via the conduit, the vacuum source configured to draw fluid from the fluid collection device.

11. A method to manufacture a fluid collection device as claimed in any one of claims 1 to 9, the method comprising:
providing a raw material mixture;
shaping the raw material mixture into a fluid impermeable barrier by injection molding the raw material, over molding the fluid impermeable barrier onto the connector.

## Patentansprüche

1. Fluidsammelvorrichtung (200), umfassend:
eine fluidundurchlässige Barriere (202), die zumindest teilweise eine Kammer (206) definiert, wobei die fluidundurchlässige Barriere auch eine sich durch sie hindurch erstreckende Öffnung definiert, wobei die Öffnung ausgebildet ist, benachbart zu einer Harnröhre eines Benutzers positioniert zu werden;
ein permeables Material, das in der Kammer angeordnet ist; und
eine Leitung (212), die einen Einlass (216) und einen Auslass einschließt, wobei der Einlass in der Kammer positioniert ist und der Auslass sich durch eine Mündung in der fluidundurchlässigen Barriere erstreckt;
und **dadurch gekennzeichnet, dass**
i) die fluidundurchlässige Barriere mindestens ein darin enthaltenes Treibmittel einschließt, wobei das mindestens eine Treibmittel zusammengesetzt ist, um eine Flexibilität der fluidundurchlässigen Barriere zu erhöhen und eine ausgewählte geometrische Konfiguration beizubehalten;
ii) weiter umfassend einen Verbinder, der innerhalb der Kammer angeordnet ist, wobei der Einlass der Leitung mit dem Verbinder gekoppelt ist, wobei der Verbinder eine Shore-Härte einschließt, die größer ist als die der fluidundurchlässigen Barriere, und **dadurch gekennzeichnet, dass**
iii) die fluidundurchlässige Barriere über den Verbinder umspritzt ist.

2. Fluidsammelvorrichtung nach Anspruch 1, wobei die fluidundurchlässige Barriere ein thermoplastisches Elastomer einschließt.

3. Fluidsammelvorrichtung nach Anspruch 2, wobei das thermoplastische Elastomer mindestens eines von Polyethylen, Polypropylen, Polyamid und Polyvinylchlorid einschließt.

4. Fluidsammelvorrichtung nach einem der Ansprüche 1-3, wobei die fluidundurchlässige Barriere weniger als 10 % des mindestens einen Treibmittels nach Volumen einschließt.

5. Fluidsammelvorrichtung nach einem der Ansprüche 1-4, wobei die fluidundurchlässige Barriere zwischen etwa 0,4 % und etwa 5 % des mindestens einen Treibmittels nach Volumen einschließt.

6. Fluidsammelvorrichtung nach einem der Ansprüche 1-5, wobei die fluidundurchlässige Barriere eine spezifische Dichte von weniger als 0,85 aufweist.

7. Fluidsammelvorrichtung nach einem der Ansprüche 1-6, wobei das mindestens eine Treibmittel ein Natriumhydrogencarbonat einschließt.

8. Fluidsammelvorrichtung nach einem der Ansprüche 1-7, wobei das mindestens eine Treibmittel eine Azidverbindung einschließt.

9. Fluidsammelvorrichtung nach einem der Ansprüche 1-8, wobei die fluidundurchlässige Barriere eine zylindrische Form mit einer sich darin in Längsrichtung erstreckenden Öffnung definiert.

10. Fluidsammelsystem, umfassend:
ein Flüssigkeitsspeichergefäß, das zum Halten einer Flüssigkeit ausgebildet ist;
eine Fluidsammelvorrichtung nach einem der vorstehenden Ansprüche, die fluidisch mit dem Fluidspeicherbehälter gekoppelt ist, und
eine Vakuumquelle, die über die Leitung mit einem oder mehreren von dem Fluidspeicherbehälter oder der Fluidsammelvorrichtung fluidisch gekoppelt ist, wobei die Vakuumquelle ausgebildet ist, Fluid aus der Fluidsammelvorrichtung zu ziehen.

11. Verfahren zum Herstellen einer Fluidsammelvorrichtung nach einem der Ansprüche 1 bis 9, wobei das Verfahren Folgendes umfasst:
Bereitstellen einer Rohmaterialmischung;
Formen der Rohmaterialmischung zu einer fluidundurchlässigen Barriere durch Spritzgießen des Rohmaterials, wobei die fluidundurchlässige Barriere über den Verbinder umspritzt wird.

## Revendications

1. Dispositif de collecte de fluide (200), comprenant :
une barrière imperméable aux fluides (202) délimitant au moins partiellement une chambre (206), la barrière imperméable aux fluides délimitant également une ouverture s'étendant à travers celle-ci, l'ouverture étant configurée pour être positionnée à proximité d'un urètre d'un utilisateur ;
un matériau perméable disposé dans la chambre ; et
un conduit (212) incluant une entrée (216) et une sortie, l'entrée étant positionnée dans la chambre et la sortie s'étendant à travers un orifice dans la barrière imperméable aux fluides ;
et **caractérisé en ce que** :
i) la barrière imperméable aux fluides inclut au moins un agent moussant incorporé dans celle-ci, l'au moins un agent moussant est conçu pour augmenter une flexibilité de la barrière imperméable aux fluides et maintenir une configuration géométrique sélectionnée
ii) comprenant en outre un raccord disposé dans la chambre, l'entrée du conduit étant accouplée au raccord, dans lequel le raccord inclut une dureté Shore supérieure à celle de la barrière imperméable aux fluides, et **en ce que**
iii) la barrière imperméable aux fluides est surmoulée sur le raccord.

2. Dispositif de collecte de fluide selon la revendication 1, dans lequel la barrière imperméable aux fluides
inclut un élastomère thermoplastique.

3. Dispositif de collecte de fluide selon la revendication 2, dans lequel l'élastomère thermoplastique
inclut au moins un parmi le polyéthylène, le polypropylène, le polyamide, et le polychlorure de vinyle.

4. Dispositif de collecte de fluide selon l'une quelconque des revendications 1 à 3, dans lequel la barrière imperméable aux fluides inclut, en volume, une quantité de l'au moins un agent moussant inférieure à 10 %.

5. Dispositif de collecte de fluide selon l'une quelconque des revendications 1 à 4, dans lequel la barrière imperméable aux fluides inclut, en volume, entre environ 0,4 % et environ 5 % de l'au moins un agent moussant.

6. Dispositif de collecte de fluide selon l'une quelconque des revendications 1 à 5, dans lequel la barrière imperméable aux fluides inclut une densité spécifique inférieure à 0,85.

7. Dispositif de collecte de fluide selon l'une quelconque des revendications 1 à 6, dans lequel l'au moins un agent moussant inclut un bicarbonate de sodium.

8. Dispositif de collecte de fluide selon l'une quelconque des revendications 1 à 7, dans lequel l'au moins un agent moussant inclut un composé azoture.

9. Dispositif de collecte de fluide selon l'une quelconque des revendications 1 à 8, dans lequel la barrière imperméable aux fluides délimite une forme cylindrique avec une ouverture s'étendant longitudinalement en son sein.

10. Système de collecte de fluide comprenant :
un récipient de stockage de fluide configuré pour contenir un fluide ;
un dispositif de collecte de fluide tel que revendiqué dans l'une quelconque des revendications précédentes, accouplé
fluidiquement au récipient de stockage de fluide et
une source de vide accouplée fluidiquement à un ou plusieurs parmi le récipient de stockage de fluide ou le dispositif de collecte de fluide par l'intermédiaire du conduit, la source de vide étant configurée pour aspirer du fluide provenant du dispositif de collecte de fluide.

11. Procédé de fabrication d'un dispositif de collecte de fluide tel que revendiqué dans l'une quelconque des revendications 1 à 9, le procédé consistant à :
fournir un mélange de matières premières ;
façonner le mélange de matières premières en une barrière imperméable aux fluides par moulage par injection de la matière première, surmouler la barrière imperméable aux fluides sur le raccord.
